# EUROPEAN PATENT APPLICATION

(11) **EP 3 989 232 A1**
(43) Date of publication of application: **27.04.2022**
(21) Application number: 20211802.2
(22) Date of filing: 04.12.2020
(51) Int. Cl.: G16H 10/40, G16H 40/40

(54) **TRANSMISSION OF ANALYSER SYSTEM DATA**

(30) Priority: 22.10.2020 IN 202011046143
(71) Applicant: Radiometer Medical ApS, 2700 Brønshøj (DK)
(72) Inventor: HASTRUP, Morten, 2700 Brønshøj (DK); RAJATHANAKODI, Rajkamal, 2700 Brønshøj (DK); LENKA, Manoj, 2700 Brønshøj (DK)
(74) Representative: Radiometer IPR

(57) **Abstract**

Disclosed is a method for transmitting data from an analyser system 1 comprising one or more analyser devices 5 to a remote server 3, wherein the one or more analyser devices 5 are configured to obtain data from samples and/or measurements taken of a patient. The method comprises determining data to be transmitted based on one or more rules associated with a geographical and/or an organizational location of the analyser system 1, and transmitting the determined data to the remote server 3.

Corresponding computer program product, analyser system, remote server, and health care system are also disclosed.

## Description

### TECHNICAL FIELD

The present disclosure relates generally to the field of transmission of data. More particularly, it relates to transmission of data from an analyser system to a remote server.

### BACKGROUND

Analyser devices are typically configured to measure one or more parameters relating to patient health and generate data used for diagnosis and monitoring of patients. Such analyser devices could, for example, comprise blood gas analysers, transcutaneous monitoring systems, and immunoassay analysers etc.

Transmission of generated data from analyser devices and/or analyser systems comprising analyser devices is typically regulated by laws and regulations per region and/or country. For example, in some regions or countries, only some of the generated data may be allowed to be transmitted from the analyser device and/or the analyser system.

A first drawback of region/country-specific regulations on transmission of analyser system data is that data transmission for analyser devices and/or analyser systems located in those regions/countries are not implemented.

A second drawback of region/country-specific regulations on transmission of analyser system data is that each analyser device may need to be customized at set-up for the concerned region/country so that only allowed data for that region/country is transmitted.

For example, a field technician or an operator may when initially setting up the analyser device and/or the analyser system need to customize the analyser device/system according to the region/country-specific regulations.

A third drawback of region/country-specific regulations on transmission of analyser system data is that any updates may require that each analyser device/system concerned be manually updated so that only allowed analyser system data for that region/country is transmitted.

For example, a field technician or an operator may when updates need to be implemented re-customize the analyser device/system according to the region/country-specific regulations.

A fourth drawback of region/country-specific regulations on transmission of analyser system data is that human-generated (e.g., by the field technician or the operator) errors may occur while customizing, re-customizing, or updating the analyser device/system.

Therefore, there is a need for alternative approaches for regulated transmission of analyser system data.

### SUMMARY

It is an object of some embodiments to solve or mitigate, alleviate, or eliminate at least some of the above or other drawbacks.

According to a first aspect, this is achieved by a method for transmitting data from an analyser system comprising one or more analyser devices to a remote server, wherein the one or more analyser devices are configured to obtain data from samples and/or measurements taken of a patient.

The method comprises determining data to be transmitted based on one or more rules associated with a geographical and/or an organizational location of the analyser system, and transmitting the determined data to the remote server.

In some embodiments, the determining of the data to be transmitted is based on one or more rules available in the analyser system.

In some embodiments, the one or more rules may beas part of software and/or as a look-up table.

In some embodiments, the one or more rules are provided to the analyser system by the remote server.

In some embodiments, the method further comprises obtaining the geographical and/or the organizational location of the analyser system.

In some embodiments, the geographical and/or the organizational location may be provided by e.g. the remote server, a certified technician or a GPS.

In some embodiments, the method further comprises providing an identity of the analyser system to the remote server for obtaining the geographical and/or the organizational location of the identified analyser system.

In some embodiments, the identity of the analyser system may be any one of: an identifier, geographical location of the analyser system, identification of rule set, the rule set, serial number, etc.

In some embodiments, the analyser system further comprises a local server implementing a point of care management system configured to provide device management of the one or more analyser devices.

In some embodiments, the remote server us connectable to one or more other remote servers.

In some embodiments, the transmission of data is performed over a packet-switched network.

In some embodiments, information from the remote server may be pushed to the analyser system without request from the analyser system, e.g., periodically, or at updates, or at each initialization etc., or the information may be provided by the remote server by request from the analyser system.

In some embodiments, the analyser system may request it's organizational location and supply information, e.g. an identity, to the remote server, which will allow the remote server to obtain the analyser system's organizational location, for example from a database, local or remote.

In some embodiments, the analyser system further comprises a local server implementing a point of care management system configured to provide device management of the one or more analyser devices, wherein communication between the analyser system and the remote server may be done via the local server.

In some embodiments, the analyser system comprises an Internet of Things (loT) Gateway.

In some embodiments, the local server comprises an loT Gateway.

In some embodiments, the remote server comprises an loT Hub.

In some embodiments, the remote server is connectable to one or more other remote servers.

In some embodiments, the transmission of data is performed over a packet-switched network such as the internet.

In some embodiments, data is obtained from analysis of one or more biological samples and/or one or more non-invasive measurements from a patient.

In following aspects, terms and features may correspond to the terms and features having the same name as in the first aspect and therefore the descriptions and explanations of the terms and features given above apply also to the following aspects.

According to a second aspect, this is achieved by a computer program product comprising a non-transitory computer readable medium, having thereon a computer program comprising program instructions, the computer program being loadable into a data processing unit and configured to cause execution of the method according to the first aspect when the computer program is run by the data processing unit.

In some embodiments, a data processing unit may be comprised in the analyser device, analyser system, and/or in the remote server(s).

According to a third aspect, this is achieved by an analyser system for transmitting data to a remote server, where the analyser system comprises one or more analyser devices and the one or more analyser devices are configured to obtain data from samples and/or measurements taken of a patient.

The analyser system is configured to determine data to be transmitted based on one or more rules associated with the geographical and/or organizational location of the analyser system, and transmit the determined data to the remote server.

In some embodiments, the analyser system is further configured to determine the data to be transmitted based on one or more rules available in the analyser system.

In some embodiments, the analyser system is further configured to obtain the one or more rules from the remote server.

In some embodiments, the analyser system is further configured to obtain the geographical and/or organizational location of the analyser system.

In some embodiments, the analyser system is further configured to provide an identity of the analyser system to the remote server, and obtain the geographical and/or the organizational location of the identified analyser system from the remote server.

In some embodiments, the analyser system further comprises a local server implementing a point of care management system configured to provide device management of the one or more analyser devices.

In some embodiments, the data is transmitted to the remote server over a packet-switched network.

In some embodiments, the packet-switched network comprises the internet.

In some embodiments, the analyser system comprises an loT Gateway.

In some embodiments, the local server comprises an loT Gateway.

In some embodiments, the data is obtained from analysis of one or more biological samples and/or one or more non-invasive measurements.

According to a fourth aspect, this is achieved by a remote server for obtaining data from an analyser system comprising one or more analyser devices, wherein the one or more analyser devices are configured to obtain data from samples and/or measurements taken of a patient.

The remote server is configured to obtain an identity of the analyser system, and in response to obtainment of the identity of the analyser system transmit, to the analyser system, a geographical and/or an organizational location, or a confirmation of the geographical and/or the organizational location of the analyser system, and/or transmit, to the analyser system, one or more rules, or a confirmation of the one or more rules, wherein the one or more rules are associated with the geographical and/or the organizational location of the analyser system.

In some embodiments, the remote server is further configured to determine the geographical and/or the organizational location of the analyser system by retrieval of the location information from a database.

In some embodiments, the database may be e.g., a data repository, or a Customer Relationship Management (CRM) system or similar, etc.

In some embodiments, the remote server is further configured to retrieve the location information from a database based on the obtained identity of the analyser system.

In some embodiments, the database may be e.g. a data repository, or a Customer Relationship Management system or similar, etc.

In some embodiments, the remote server is further configured to perform the acts of obtainment and transmittal at least during an initial setup of the analyser system or continuously during up-time of the analyser system.

In some embodiments, the transmission of data is performed over a packet-switched network.

In some embodiments, the packet-switched network may be the internet.

In some embodiments, the remote server comprises an loT hub.

In some embodiments, the remote server is further configured to communicate with an loT Gateway comprised in the analyser system.

In some embodiments, the remote server is connectable to one or more other remote servers.

According to a fifth aspect, this is achieved by a health care system comprising an analyser system and a remote server configured to perform the steps of the method according to the first aspect.

In some embodiments, the analyser system may be an analyser system according to the third aspect.

In some embodiments, the remote server may be a remote server according to the fourth aspect.

Any of the above aspects may additionally have features identical with or corresponding to any of the various features as explained above for any of the other aspects.

An advantage of some embodiments is that alternative approaches for regulated transmission of analyser system data are provided.

An advantage of some embodiments is the provision of automatic customization of analyser device/system for transmission of analyser system data according to the region/country-specific regulations.

An advantage of some embodiments is the enablement of remote customization of analyser device/system for transmission of analyser system data according to the region/country-specific regulations.

An advantage of some embodiments is the provision of automatic updating of the analyser device/system for transmission of analyser system data according to the region/country specific regulations.

An advantage of some embodiments is the enablement of remote updating of the analyser device/system for transmission of analyser system data according to the region/country specific regulations.

An advantage of some embodiments is the provision of a reliable system for following region/country specific regulations on transmission of analyser system data.

An advantage of some embodiments is that by the remote server being connectable to other remote servers the transmitted data from the analyser devices, which is received at the remote server, may be further transmitted to other remote servers for processing, secure storage, retention etc.

An advantage of some embodiments is that by updating the rules set regularly or continuously any new regulations on transmission of data may be reflected in the updated one or more rules.

An advantage of some embodiments is that human-generated (e.g., by the field technician or the operator) errors that may occur while customizing, re-customizing, or updating the analyser device/system are reduced by the automatic customization, re-customization, or updating of analyser device/system.

An advantage of some embodiments is that by certificate provisioning in the initial setup, wherein the remote server provides a certificate to the analyser system, the analyser system is trusted with transmission of data to the remote server. In the case that the analyser system is compromised in any way, the provided certificate may be revoked so that the analyser system no longer is trusted with transmission of data to the remote server.

An advantage of some embodiments is that by the remote server being connectable to other remote servers is that transmitted data from the analyser system, which is received at the remote server, may be further transmitted to other remote servers for processing, secure storage, retention etc.

### BRIEF DESCRIPTION OF THE DRAWINGS

In the following, example embodiments are described in more detail with reference to the accompanying drawings, wherein:
Figure 1 is a flowchart illustrating example method steps according to some embodiments;
Figure 2 is a flowchart illustrating example method steps according to some embodiments;
Figure 3 is a flowchart illustrating example method steps according to some embodiments;
Figure 4 is a flowchart illustrating example method steps according to some embodiments;
Figure 5 is a flowchart illustrating example method steps according to some embodiments;
Figure 6 is a flowchart illustrating example method steps according to some embodiments;
Figures 7A-B are schematic drawings illustrating data transmission between an example analyser system and example remote server system according to some embodiments;
Figures 8A-B are schematic drawings illustrating data transmission between an example analyser system and example remote server system according to some embodiments;
Figures 9-10 are schematic drawings illustrating data transmission between an example analyser system and example remote server system according to some embodiments; and
Figures 11-12 are schematic drawings illustrating example health care systems according to some embodiments.

### DETAILED DESCRIPTION

Embodiments of the present disclosure will be described and exemplified more fully hereinafter with reference to the accompanying drawings. The description disclosed herein can, however, be realized in many different forms and should not be construed as being limited to the embodiments set forth herein.

As mentioned above, consequences of region/country-specific regulations on transmission of analyser system data may entail that data transmission is not implemented in analyser devices and/or analyser systems located in these regions/countries.

Also as mentioned above, a field technician or an operator may when initially setting up or updating the analyser device and/or the analyser system need to customize/re-customize the analyser device/system according to the region/country-specific regulations so that only allowed data for that region/country is transmitted.

In the following, embodiments will therefore be presented where alternative approaches for regulated transmission of analyser system data are described.

Analyser devices, as described herein, may typically comprise devices configured to generate health related data and/or device data in response to analysis of samples, e.g., biological samples, and/or measurements, e.g., transcutaneous measurements, taken of a patient.

Analyser devices, as mentioned above, may therefore be configured to measure one or more parameters relating to patient health, generate data, produce test results used for diagnosing and monitoring patients.

Analyser devices may comprise, for example, blood gas analysers, transcutaneous monitoring systems, immunoassay analysers, and the measurements performed could be, e.g., on biological samples, such as blood samples, or they could be ,e.g., non-invasive measurements, such as transcutaneous measurements. The generated data from samples and/or measurements taken of a patient may be obtained at a point of care, or, alternatively or additionally, obtained from samples and/or measurements taken of/from the patient at a laboratory.

Additionally, an analyser device may be a sophisticated apparatus that must perform to a high standard and so need to be frequently monitored to ensure proper functioning so that any errors are dealt with swiftly. As part of the monitoring of an analyser device, device data, i.e. meta data, may be generated, for example, when maintenance is performed, at quality check results, at software version updates, during up-time of the analyser, etc.. The device data may be generated by the analyser device to aid in keeping the device up and running within specifications.

Analyser devices may, in some embodiments, be comprised in an analyser system.

Analyser systems, as described herein, may typically comprise a management system configured to provide device management of one or more analyser devices and wherein the device management may comprise a centralized data management system.

Alternatively or additionally, the management system may be implemented in a local server operably connected to the or more analyser devices of the analyser system.

In some analyser systems, comprising one or more analyser devices, the data generated or produced by the analyser device(s), such as the test results and/or device data as described above, is transmitted to a remote server. The remote server may then provide a number of services in response to the transmitted data. For example, the remote server may aid in the analysis of test results by collecting, modelling and interpreting the data, possibly even in real-time, to produce additional information for a medical professional, e.g., an operator of the analyser device, based on the test results. Further, the remote server may offer technical support for the analyser device by collecting device data, and possibly also test results, to monitor and detect any technical errors in the analyser device and offer assistance such as, e.g., remote support and troubleshooting, software updates, etc. In such a setup, as a general rule, patient or patient ID-related data are typically not visible or accessible at any time.

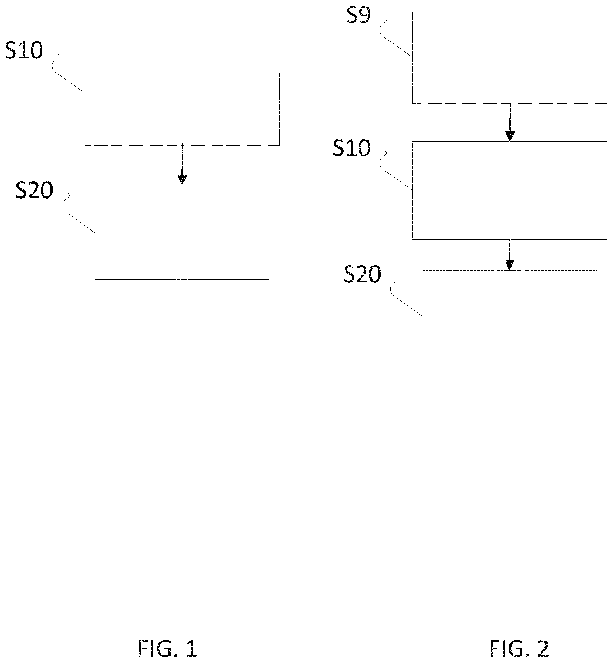

Figures 1-6 are flowcharts illustrating method steps of example methods for transmitting data from an analyser system, which comprises one or more analyser devices, to a remote server.

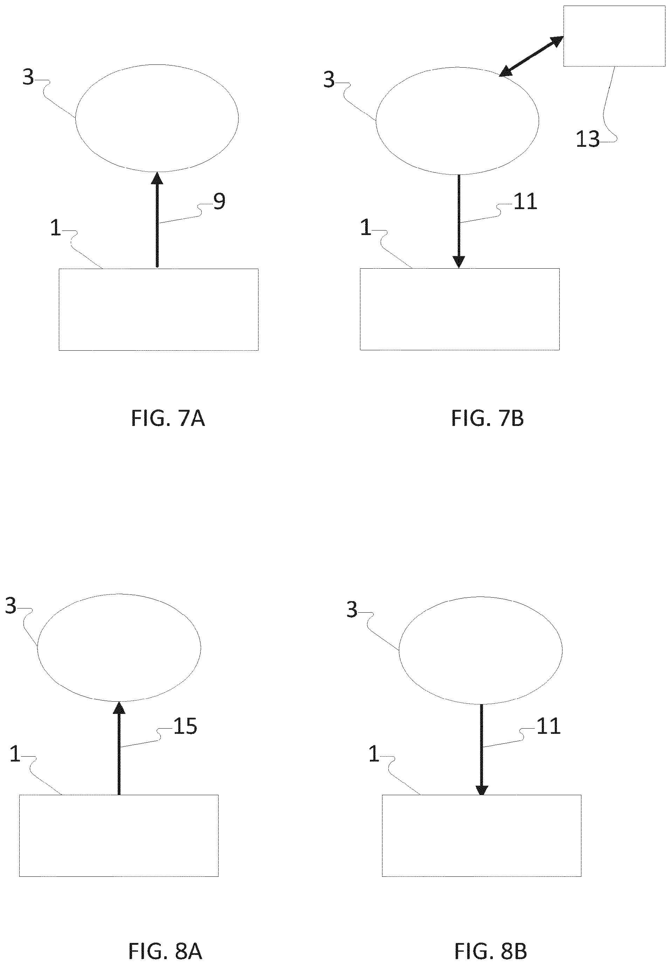

Figures 7-10 are schematic drawings illustrating examples of analyser systems 1 exchanging data with a remote server 3.

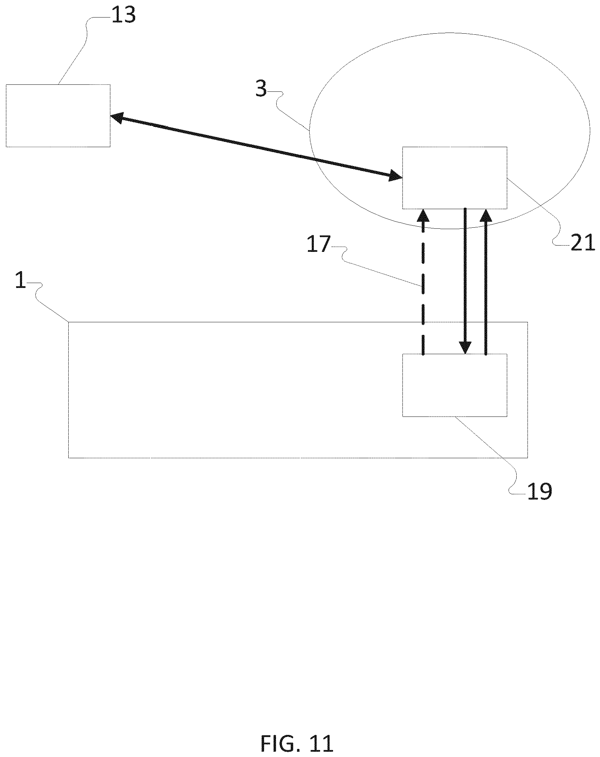

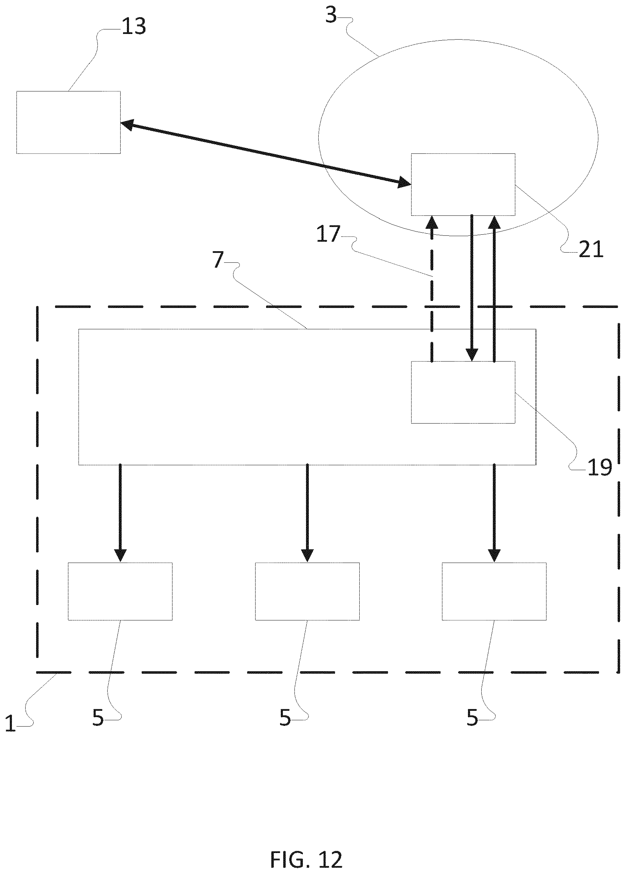

Figures 11-12 are schematic drawings illustrating examples of health care systems. An analyser system 1 comprising several analyser devices 5 and a local server 7 is shown in fig. 12, where the local server may implement a point of care management system configured to provide device management of the one or more analyser devices 5.

The analyser devices 5 are devices that are configured to analyse samples and/or measurements taken of a patient and thereby measure parameters related to the patient's health and thereby produce or generate data (also referred to as health related data in here), e.g., test results, used in diagnosis and monitoring of patients. Further, the analyser devices 5 generate device data, e.g., maintenance related data, which is used to optimize and maintain the functionality of the analyser device 5. The generated health related data and/or the device data may be data for transmission to the remote server 3. The data for transmission to the remote server 3 may be subject to location-specific regulations dictating in terms of rules on what may be transmitted from the analyser system 1 to the remote server 3.

Therefore, in step S10, a determination is made of the data to be transmitted based on one or more rules, where the rules are constructed so as to make the data transmission comply with local regulations, e.g., the application of the one or more rules may limit the data such that a type of data is not included in the transmission, or it may allow all the data in a given transmission. Limiting data for transmission may mean that the data not permitted to be transmitted is filtered away before the transmission. Thus, determining data to be transmitted may comprise filtering or selecting data. The local regulations may be specific to a region or a country and the set rules define what type of data can be transmitted, wirelessly or via a cabled network, in particular via a packet-switched network such as the Internet, from the analyser system 1 to the remote server 3, according to regulations in force at the location of the analyser system.

To reflect the local regulations, the one or more rules are associated with a geographical or organizational location, where the organizational location refers to the subsidiary structure in an organizational structure to which the analyser system 1 belongs. The subsidiary structure is used in a specific geographical region, such as a country. For example, the analyser system 1 could belong to an organizational subsidiary structure, which all analyser devices in e.g. France, or Germany, or China, etc. belong to. In this way, the organizational location is another way of linking the analyser system 1 with an appropriate set of rules for transmission of data. The geographical location may be GPS coordinates, a city, a region, a country, etc. That the one or more rules are associated with a geographical and/or an organizational location means that the rules are formulated so as to be specific for a geographical and/or an organizational location. Thus, the one or more rules can be used to control the transmission of data from the analyser system to the remote server according to regulations in force at the location of the analyser system.

In step S20, the determined data is transmitted to the remote server 3. The remote server 3 may be located, at least partly, in the same region or country as the analyser system 1 or it may be located, at least partly, outside the region or country of the analyser system 1. The remote server may be a cloud server such that the transmitted data may be stored at any data centre acting as server space for the cloud server. Further, the remote server 3 could be connectable to one or more other remote servers. When the remote server 3 is connected to other remote servers, the transmitted data from the analyser devices may be further transmitted from the remote server 3 to the connected remote servers for processing, secure storage, retention of the transmitted data etc.

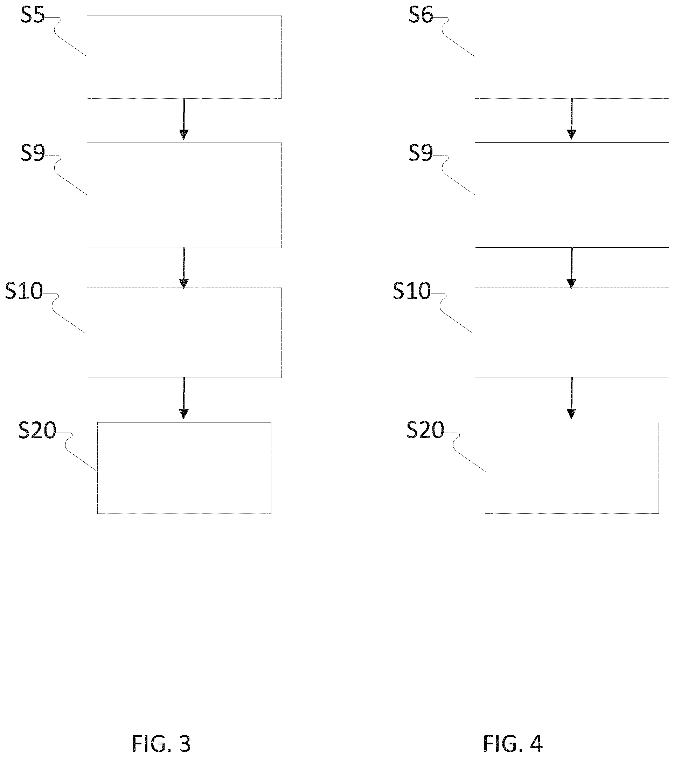

The rules set for determining the data to be transmitted, which may be implemented at the analyser system 1, may be obtained by the analyser system 1 in a number of ways, for example, the one or more rules may be obtained from the remote server 3, reference to step S9 illustrated in Figure 2. As illustrated in Figure 3, the remote server 3 may provide the one or more rules after a step S5, wherein the analyser system 1 provides information on its identity to the remote server 3. This is also illustrated in Figures 7A and 7B. In Figure 7A, the analyser system 1 sends identity information 9 to the remote server 3. The identity information may be one of an identifier, geographical and/or organizational location of the analyser system, identification of rule set, the rule set, serial number, etc. In response to receiving the identity information 9, the remote server 3 provides the analyser system 1 with one or more rules 11. The remote server 3 may consult a Customer Relationship Management (CRM) system 13 operably connected to the remote server 3 or other similar system to obtain, for example, the geographical and/or the organizational location of the analyser system 1. Due to the heavy regulations on analyser devices 5, a CRM or other similar system operated by the manufacturer will have information on the location in which an analyser device 5 is to be installed.

Alternatively, or additionally, as illustrated in Figure 4, in step S6, the analyser device 1 may provide information on its geographical and/or organizational location 15 to the remote server 3 to which the remote server 3 responds by providing a rules set 11 to the analyser system 1. This is also illustrated schematically in Figures 8A and 8B. In fig. 8A, the analyser system 1 sends location information 15 to the remote server 3 and after receiving the location information 15, the remote server 3 provides the analyser system 1 with one or more rules 11.

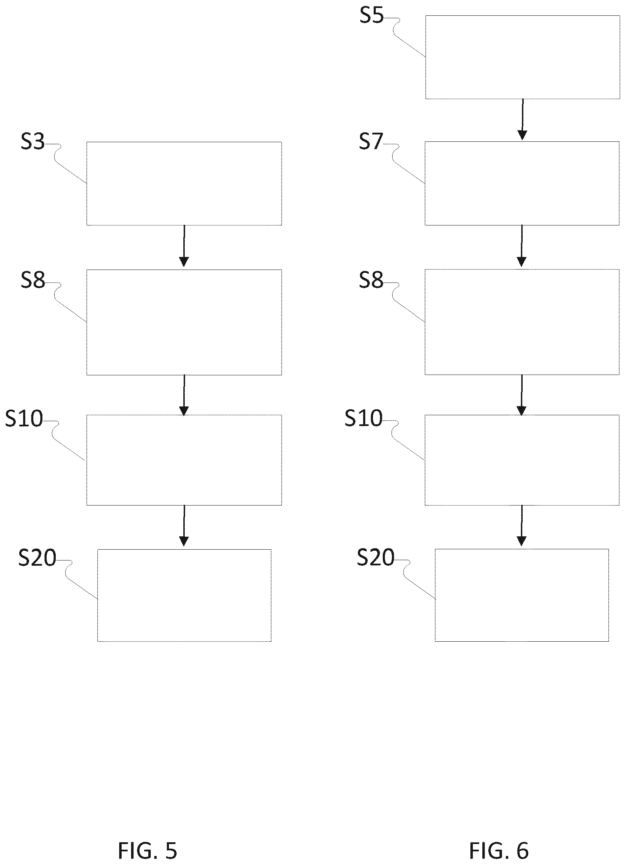

The rules set may be based on one or more rules available in the analyser system 1. That is, the analyser system 1 comprises the rules set, for example as a look-up table. The analyser system 1 may be configured to generate the rules set after obtaining its geographical and/or organizational location for example from the remote server 3, from a certified technician or from a GPS, as illustrated in Figure 5. In step S3 in Figure 5, the analyser system 1 obtains its geographical and/or organizational location and in step S8, the analyser system 1 generates the rules set on the basis of the location information. For example, a certified technician could provide the analyser system 1 with information about its geographical location and/or it's organizational location, possibly during an initial setup of the analyser system 1. As another example, the analyser system 1 could comprise a GPS to provide it with its geographical location. The rules set may be generated by selecting the rules set from a database, which could be updated regularly, for example by updates being transmitted from the remote server 3 to the analyser system 1.

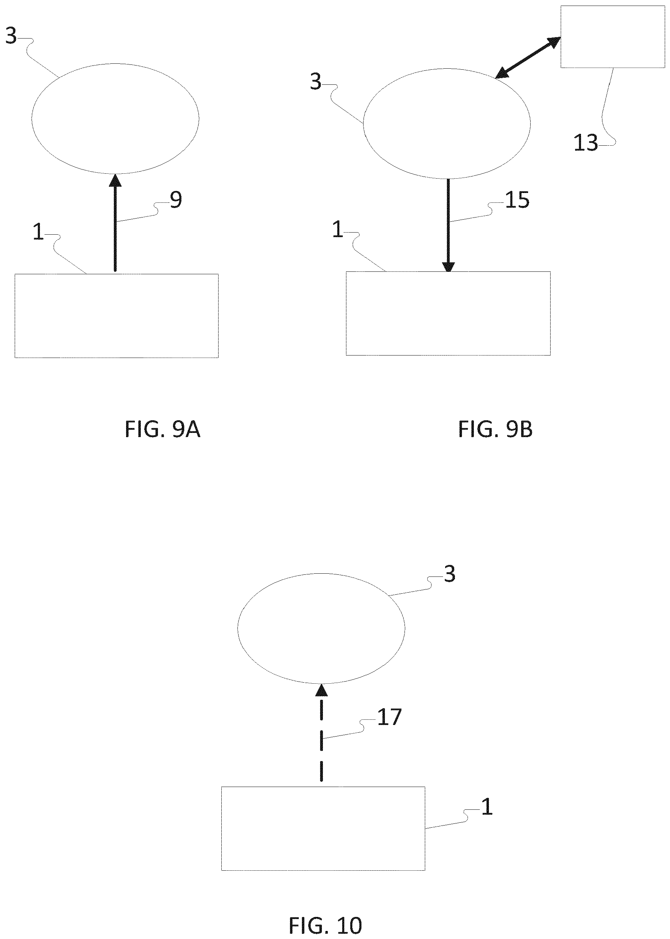

In step S5 as illustrated in Figure 6, the analyser system 1 provides information on its identity to the remote server 3 to obtain, in step S7, its location from the remote server 3, or as a confirmation of the location if the analyser system 1 has already been provided with its geographical and/or the organizational location. After being provided with its location information, or confirmation, the analyser system 1 generates the one or more rules in step S8 as illustrated in Figure 5. This is further illustrated schematically in Figures 9A and 9B, where in Figure 9A the analyser system 1 provides information on its identity 9 to the remote server 3. The remote server 3 then provides the geographical and/or the organizational location 15 of the analyser system 1 in response to identifying the analyser system 1. Again, the remote server 3 may consult a data repository, a Customer Relationship Management (CRM) system 13 or other similar system to obtain the geographical and/or organizational location of the analyser system 1.

The obtainment by the analyser system 1 of a rules set used to determine data to be transmitted to the remote server 3, may be performed at least during an initial setup of the analyser system 1 or continuously during up-time of the analyser system 1. By obtaining a rules set regularly or continuously, the rules set can be updated and any new regulations can be reflected in the updated one or more rules. The initial setup may be a provisioning, wherein the remote server provides a certificate to the analyser system, which allows the analyser system to send data to the remote server. If the analyser system later becomes compromised in any way, the certificate can be revoked such that the analyser system can no longer send data to the remote server.

Generally, for all embodiments, information from the remote server 3 may be pushed to the analyser system 1 without request from the analyser system, e.g., periodically, or at updates, or at each initialization etc., or the information may be provided by the remote server 3 by request from the analyser system 1. As illustrated in Figure 10, after obtaining the one or more rules, the analyser system 1 is able to transmit the determined data 17 (shown as a dashed line), which may be limited data, for example such that a type of data is not included in the transmission, or it may allow all the data in a given transmission.

The method for transmission of data from an analyser system may be executed by a computer program product, which comprises a non-transitory computer readable medium on which a computer program comprising program instructions is stored. The computer program can be loaded into a data processing unit and configured to cause execution of the method, when the computer program is run by the data processing unit. The data processing unit could be a part of the analyser device, analyser system, and/or in the remote server(s).

Figure 11 illustrates an example of a health care system 110, which comprises an analyser system 1 and a remote server 3, and is configured to perform the method (method steps as illustrated and described in connection with Figures 1-6) for transmitting data from an analyser system 1, which may include one or more analyser devices 5, to a remote server 3. The analyser system 1 may be located at a user site and comprise an Internet of Things (IoT) Gateway 19, which it uses for communication with the remote server 3 via an IoT Hub 21 comprised in or operably connected to the remote server 3. Thus, the interconnection via, e.g. the Internet, of the analyser system and the remote server enables them to send and receive data. IoT devices may be identified with a unique identifier (UID) and enables transfer of data over a network without requiring human-to-human or human-to-computer interaction. The loT device is assigned an Internet Protocol (IP) address and is thereby able to transfer data over a network enabling the communication needed between the analyser system and the remote hub. The loT Hub 21 may further communicate with a CRM 13 or other similar system from which the remote server 3 can obtain information related to the specific analyser system 1 at the user site. The analyser system 1 obtains one or more rules associated to the location of the analyser system 1 (geographical and/or organisational location), which it applies before transmitting any type of data, such as health related data and device data, to the remote server 3.

As an example, in the health care system illustrated in Figure 11, the analyser system 1 can transmit, to the remote server 3, its identity information (reference to Figure 9A). The remote server can then look up the analyser system id 9 in the CRM 13 or other similar system to learn the organizational location, also sometimes referred to as a business unit, of the analyser system 1. This information, i.e. the organizational location, can then be provided to the analyser system 1 via the IoT Gateway 19 , wherein software of the analyser system 1 is configured to determine, from its provided organizational location, which rules are mandated in the country or region in which it is located. The analyser system 1 can then apply one or more rules to ensure that its transmission of data to the remote server 3 is implemented according to local regulations.

Figure 12 illustrates another example of a health care system 120, which comprises an analyser system 1 and a remote server 3, and is configured to perform the method (method steps as illustrated and described in connection with Figures 1-6) for transmitting data from an analyser system 1, which may include one or more analyser devices 5, to a remote server 3. In addition to the components of the health care system as illustrated in Figure 11, the analyser system 1 may also include a local server 7 implementing a point of care management system that is configured to provide device management of, in this example system, three analyser devices 5.

### LIST OF REFERENCES

- 1: Analyser system
- 3: Remote server
- 5: Analyser device
- 7: Local server
- 9: Identity information transmitted
- 11: Rule set transmitted
- 13: CRM
- 15: Geographical and/or organizational location transmitted
- 17: Determined data transmitted
- 19: loT Gateway
- 21: IoT Hub

## Claims

1. A method for transmitting data from an analyser system (1) comprising one or more analyser devices (5) to a remote server (3), wherein the one or more analyser devices (5) are configured to obtain data from samples and/or measurements taken of a patient, the method comprising the steps of:
- determining data to be transmitted based on one or more rules associated with a geographical and/or an organizational location of the analyser system (1), and
- transmitting the determined data to the remote server (3).

2. The method according to claim 1, wherein the determining of the data to be transmitted is based on one or more rules available in the analyser system (1).

3. The method according to claim 2, wherein the one or more rules are provided to the analyser system (1) by the remote server (3).

4. The method according to any of claims 1-3, further comprising the step of:
- obtaining the geographical and/or the organizational location of the analyser system (1).

5. The method according to any of claims 1-4, further comprising the step of:
- providing an identity of the analyser system (1) to the remote server (3) for obtaining the geographical and/or the organizational location of the identified analyser system (1).

6. The method according to any of the previous claims, wherein data is obtained from analysis of one or more biological samples and/or one or more non-invasive measurements from a patient.

7. A computer program product comprising a non-transitory computer readable medium, having thereon a computer program comprising program instructions, the computer program being loadable into a data processing unit and configured to cause execution of the method according to any of claims 1-6 when the computer program is run by the data processing unit.

8. An analyser system for transmitting data to a remote server, the analyser system (1) comprising one or more analyser devices (5), wherein the one or more analyser devices (5) are configured to obtain data from samples and/or measurements taken of a patient, the analyser system 1 being configured to:
- determine data to be transmitted based on one or more rules associated with the geographical and/or organizational location of the analyser system (1), and
- transmit the determined data to the remote server (3).

9. The analyser system according to claim 8, wherein the analyser system (1) is further configured to determine the data to be transmitted based on one or more rules available in the analyser system (1).

10. The analyser system according to any of claims 8-9, wherein the analyser system (1) is further configured to:
- provide an identity of the analyser system (1) to the remote server (3), and
- obtain the geographical and/or the organizational location of the identified analyser system (1) from the remote server (3).

11. The analyser system according to any of claims 8-10, wherein the analyser system 1 further comprises a local server (7) implementing a point of care management system configured to provide device management of the one or more analyser devices (5).

12. A remote server for obtaining data from an analyser system comprising one or more analyser devices (5), wherein the one or more analyser devices (5) are configured to obtain data from samples and/or measurements taken of a patient, the remote server (3) being configured to:
- obtain an identity of the analyser system (1), and
- in response to obtainment of the identity of the analyser system (1):
o transmit, to the analyser system (1), a geographical and/or an organizational location, or a confirmation of the geographical and/or the organizational location of the analyser system (1), and/or
o transmit, to the analyser system (1), one or more rules, or a confirmation of the one or more rules, wherein the one or more rules are associated with the geographical and/or the organizational location of the analyser system (1).

13. The remote server according to claim 12, wherein the remote server (3) is further configured to determine the geographical and/or the organizational location of the analyser system (1) by retrieval of the location information from a database.

14. The remote server according to claim 13, wherein the remote server (3) is further configured to retrieve the location information from a database based on the obtained identity of the analyser system (1).

15. The remote server according to any of claims 12-14, wherein the remote server (3) is further configured to perform the acts of obtainment and transmittal at least during an initial setup of the analyser system (1) or continuously during up-time of the analyser system (1).

16. A health care system comprising an analyser system (1) and a remote server (3) configured to perform the steps of the method according to any one of claims 1-6.
